# EUROPÄISCHE PATENTSCHRIFT

(11) **EP 1 192 905 B1**
(45) Veröffentlichungstag und Bekanntmachung des Hinweises auf die Patenterteilung: **10.11.2010**
(21) Anmeldenummer: 01121134.9
(22) Anmeldetag: 04.09.2001
(51) Int. Cl.: A61B 17/02

(54) **Chirurgischer Retraktor**
Surgical retractor
Ecarteur chirurgical

(30) Priorität: 02.10.2000 DE 10048790
(43) Veröffentlichungstag der Anmeldung: 03.04.2002
(73) Patentinhaber: Aesculap AG, 78532 Tuttlingen (DE)
(72) Erfinder: Cistac, Christian, 85016 La Roche sur Yon (FR); Siebert, Werner E., Wilhelmshöher Allee 345 34131 Kassel (DE); Delecrin, Joel, 44093 Nantes Cedex 01 (FR); Merz, Thomas, 73663 Berglen (DE)
(74) Vertreter: Hoeger, Stellrecht & Partner Patentanwälte

(56) Entgegenhaltungen:
- EP-A- 0 951 868
- WO-A-96/02195
- WO-A-98/27869
- WO-A-99/09892
- WO-A-99/15069
- DE-A- 19 844 251
- US-A- 4 989 587
- US-A- 5 697 891

## Beschreibung

Die Erfindung betrifft eine Vorrichtung zur Schaffung eines perkutanen Zugangs in einen Körper mit zwei gemeinsam einen Zugangskanal bildenden, jeweils ein proximales und ein distales Ende aufweisenden Retraktorblättern, deren Abstand voneinander unter Veränderung des Querschnitts des Zugangskanals veränderbar ist.

Zur Schaffung eines perkutanen Zugangs, wie er beispielsweise bei einer lumbalen Diskektomie benötigt wird, ist es bekannt, in den Körper rohrförmige Kanülen einzusetzen und an diesen mittels eines Positioniertellers oder mittels einer Positionierhülse zusätzliche Instrumente so anzuordnen, daß ein rohrschaftförmiges Teil dieser Instrumente im Randbereich des Querschnitts des Zugangskanals in diesen eintaucht (DE 198 25 763 A1; WO 97/34 537). Es ist dadurch möglich, den Rohrschaft des eintauchenden Instrumentes, beispielsweise den Rohrschaft eines Endoskops, längs des Randes des Zugangskanals in unterschiedlichen Positionen anzuordnen, wobei neben dem Rohrschaft noch ein Teil des Querschnitts des Zugangskanals frei bleibt für die Einfuhr weiterer Instrumente. Voraussetzung für eine solche Konstruktion ist jedoch eine rohrförmige Ausbildung des Zugangskanals, da nur dann ein Positionierteller oder eine Positionierhülse verdrehbar an dem Zugangskanal gelagert werden können.

Dokument WO 99/09892 offenbart eine Vorrichtung nach dem Oberbegriff des Anspruchs 1.

Andererseits ist es auch bekannt, zur Schaffung eines perkutanen Zugangs zwei gemeinsam den Zugangskanal bildende Retraktorblätter zu verwenden, die in ihrem Abstand so veränderbar sind, daß dadurch der Querschnitt des Zugangskanals veränderbar ist. Eine Lagerung von zusätzlichen Instrumenten über einen Positionierteller oder eine Positionierhülse ist bei diesen gegeneinander bewegbaren Retraktorblättern nicht möglich.

Es ist Aufgabe der Erfindung, eine gattungsgemäße Vorrichtung zur Schaffung eines perkutanen Zugangs so auszubilden, daß auch bei gegeneinander bewegbaren Retraktorblättern der Rohrschaft eines Instrumentes an unterschiedlichen Positionen des Zugangskanals angeordnet werden kann.

Diese Aufgabe wird bei einer Vorrichtung der eingangs beschriebenen Art erfindungsgemäß dadurch gelöst, daß zumindest an einem Retraktorblatt an dessen proximalem Ende eine Führung angeordnet ist, auf der ein Halter für ein einen Rohrschaft aufweisendes Instrument derart in einer quer zum Zugangskanal angeordneten Ebene verschiebbar gelagert ist, daß ein am Halter gehaltenes Instrument mit seinem Rohrschaft an verschiedener Stelle des Querschnitts des Zugangskanals in diesen eintaucht.

Es wird also an einem Retraktorblatt eine Führung angeordnet, die eine Verschiebung des Halters in einer Ebene ermöglicht, die quer zur Längsrichtung des Zugangskanals verläuft, und dies ermöglicht die Verschiebung des in den Zugangskanal eintauchenden Rohrschaftes.

Besonders vorteilhaft ist es, wenn beide Retraktorblätter jeweils eine derartige Führung für einen Halter aufweisen.

Günstig ist es, wenn die Führungen parallel zur Kontur der Retraktorblätter verlaufen. Dann ist es möglich, durch Verschiebung des Halters längs dieser Führungen den Rohrschaft längs den Retraktorblättern zu bewegen, beispielsweise unmittelbar an der Wand des Retraktorblattes entlang.

Bei einer bevorzugten Ausführungsform ist vorgesehen, daß die Führungen der beiden Retraktorblätter bei maximal angenäherten Retraktorblättern den Zugangskanal ringförmig umgeben. Dadurch ist es einmal möglich, die Halterung auf den beiden Führungen in jeder Position längs des Retraktorblattes anzuordnen, zum anderen kann bei einander angenäherten Retraktorblättern die Halterung von der Führung eines Retraktorblattes auf die Führung des anderen Retraktorblattes geschoben werden.

Insbesondere verlaufen die Führung bzw. die Führungen kreisbogenförmig.

Die Führung bzw. die Führungen können beispielsweise als in proximaler Richtung von den Retraktorblättern abstehende Leisten ausgebildet sein.

Günstig ist es, wenn der Halter eine Fixiereinrichtung aufweist, mittels der er relativ zu der Führung lösbar fixierbar ist.

Insbesondere kann die Fixiereinrichtung eine Klemmeinrichtung sein.

Es ist weiterhin vorteilhaft, wenn die Führung und der Halter zusammenwirkende Rastelemente tragen, durch die der Halter gerastert längs der Führung verschiebbar ist. Der Operateur hat dadurch die Möglichkeit, den Halter längs der Führung gerastert zu verschieben und die Position durch die Rasterung vorläufig festzulegen, eine endgültige Festlegung erfolgt durch die Fixiereinrichtung, beispielsweise eine Klemmeinrichtung.

Die Rastelemente können Vertiefungen und elastisch in diese eingreifende Vorsprünge sein.

Bei einer bevorzugten Ausführungsform ist vorgesehen, daß der Halter eine Halterung für das Instrument trägt, die relativ zum Halter in Richtung auf den Mittelpunkt des Zugangskanals verschiebbar ist. Dadurch kann zusätzlich die Position des Rohrschaftes im Inneren des Zugangsquerschnittes in Bezug auf den Mittelpunkt des Zugangskanales verändert werden, insgesamt ermöglichen diese Ausgestaltungen also, den Rohrschaft an beliebiger Stelle des Zugangskanals zu positionieren.

Die Halterung kann beispielsweise an einem Schlitten angeordnet sein, der an einer Führung des Halters verschiebbar gelagert ist.

Auch hier ist es günstig, wenn die Halterung relativ zum Halter in verschiedenen Positionen lösbar fixierbar ist.

Halter und Halterung können durch elastisch zusammenwirkende Rastelemente gerastert gegeneinander verschiebbar sein.

Am Halter kann eine Spannvorrichtung zur lösbaren Fixierung des Instrumentes am Halter angeordnet sein, so daß die Winkelstellung des Instrumentes und/oder die Eintauchtiefe des Instrumentes relativ zum Halter festgelegt werden kann.

Diese Spannvorrichtung weist bei einer bevorzugten Ausführungsform der Erfindung zwei nebeneinander den Rohrschaft des Instrumentes umgebende Halteglieder auf, die in einer quer zur Längsachse des Rohrschaftes verlaufenden Ebene gegeneinander verschiebbar sind. Dadurch wird der Rohrschaft beim Verschieben dieser Halteglieder jeweils einseitig gegen eines der Halteglieder gepreßt und im Klemmsitz zwischen ihnen gehalten.

Die Retraktorblätter können im Querschnitt bogenförmig ausgebildet sein und in seitliche Randbereiche auslaufen, die parallel zu ihrer Verschiebung beim Verändern des gegenseitigen Abstands verlaufen und sich bei angenäherten Retraktorblättern überdecken. Dadurch ist es möglich, auch bei einem größeren Abstand der Retraktorblätter einen Zugangskanal zu schaffen, der allseits von den Retraktorblättern umgeben und damit geschlossen ist.

Bei einer bevorzugten Ausführungsform werden die Retraktorblätter an gegeneinander bewegbaren und dadurch den Abstand der Retraktorblätter voneinander verändernden Tragelementen gehalten.

Diese können insbesondere zwei Haltearme sein, die schwenkbar miteinander verbunden sind und an ihren freien Enden jeweils ein Retraktorblatt tragen.

Diese Tragelemente können ihrerseits an einer ortsfesten Rahmenstruktur festlegbar sein.

Es ist vorteilhaft, wenn die Tragelemente durch eine Feststellvorrichtung in festem Abstand zueinander fixierbar sind.

Die Retraktorblätter können an den Tragelementen lösbar gehalten sein, so daß es möglich ist, für die spezielle Operation geeignete Retraktorblätter an den Tragelementen zu befestigen, beispielsweise kann ein Satz von unterschiedlich langen Retraktorblättern verwendet werden, aus dem der Chirurg beim Einsetzen der Vorrichtung das Retraktorblatt mit der richtigen Länge auswählt. Es ist auch möglich, die Retraktorblätter an den Tragelementen auszutauschen, beispielsweise beim Ändern der Operationsrichtung.

Um diese Lösbarkeit zu erreichen, kann es beispielsweise vorgesehen sein, daß zur lösbaren Festlegung der Retraktorblätter an den Tragelementen zusammenwirkende elastische Rastelemente vorgesehen sind.

Am Halter können beliebige Instrumente gehalten werden, die ein rohrschaftförmiges, in den Zugangskanal eintauchendes Teil aufweisen, insbesondere kann ein solches Instrument ein Endoskop sein.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht einer zwei Retraktorblätter aufweisenden Vorrichtung zur Schaffung eines perkuta- nen Zugangs mit einem in einen Halter eingesetzten Endoskop;
- Figur 2:: eine perspektivische Ansicht der Vorrichtung der Figur 1 ohne Endoskop und mit voneinander getrennten Einzel- teilen;

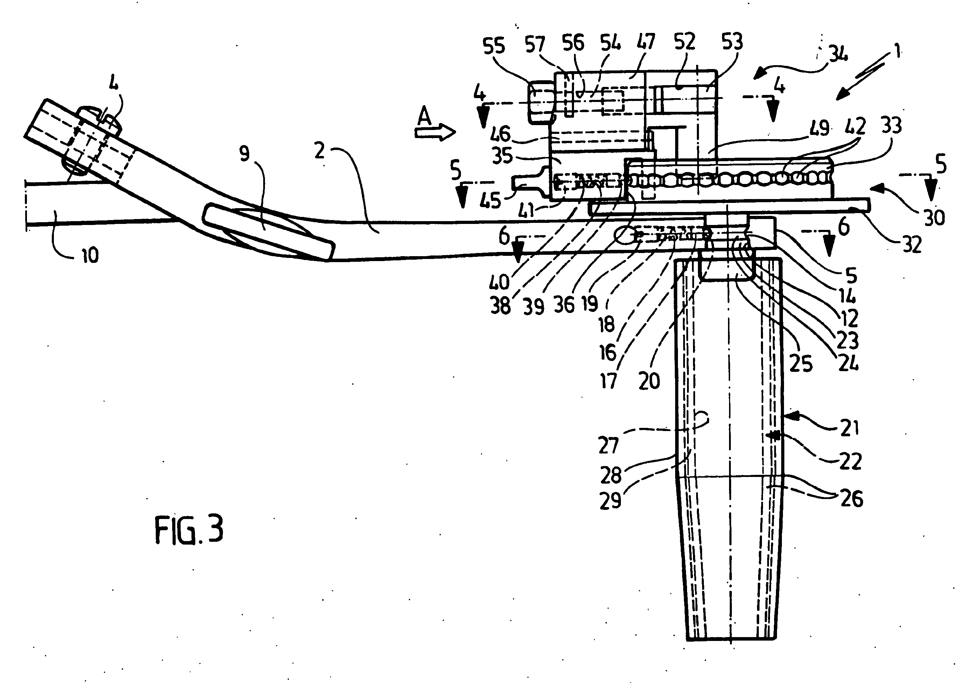

- Figur 3:: eine Seitenansicht der Vorrichtung der Figur 1 ohne En- doskop;
- Figur 4:: eine Schnittansicht längs Linie 4-4 in Figur 3;
- Figur 5:: eine Schnittansicht längs Linie 5-5 in Figur 3;
- Figur 6:: eine Schnittansicht längs Linie 6-6 in Figur 3 und
- Figur 7:: eine Teilansicht der Vorrichtung der Figur 3 in Richtung des Pfeiles A.

Die in der Zeichnung dargestellte Vorrichtung 1 zur Schaffung eines perkutanen Zugangs in einen Körper umfaßt zwei Haltearme 2, 3, die an einem Ende durch eine Lagerschraube 4 schwenkbar miteinander verbunden sind. Die Haltearme 2 und 3 sind etwa im Abstand von einem Drittel ihrer Länge von der Lagerstelle entfernt um etwa 30° abgebogen, so daß die durch die Haltearme 2, 3 im Bereich der Lagerschraube 4 aufgespannte Ebene gegenüber der Ebene, die die Haltearme 2, 3 im Bereich ihrer freien Enden 5, 6 aufspannen, um 30° geneigt ist. Im Übergangsbereich der beiden Ebenen ist eine in einem Haltearm 2 frei verdrehbar und axial unverschieblich gelagerte Verstellschraube 7 angeordnet, die eine Gewindebohrung 8 in dem anderen Haltearm durchsetzt und die mittels eines Griffes 9 verdreht werden kann, so daß dadurch der Öffnungswinkel der beiden Haltearme 2, 3 bleibend veränderbar ist.

Die Haltearme 2, 3 sind mittels einer Stange 10 an einer in der Zeichnung nur strichpunktiert angedeuteten ortsfesten Rahmenstruktur 11 festgelegt und können auf diese Weise dauerhaft so positioniert werden, daß ihre freien Enden 5, 6 über dem vorgesehenen Operationsbereich angeordnet werden können.

Beide freie Enden 5, 6 der Haltearme 2, 3 weisen eine nach außen hin offene, im Querschnitt U-förmige Aufnahmeöffnung 12 bzw. 13 auf mit einer längs der gesamten Seitenwand der Aufnahmeöffnung 12, 13 verlaufenden, mittigen vorspringenden Halterippe 14 bzw. 15.

In jedem Haltearm befindet sich eine Durchgangsbohrung 16, die im Bereich der Halterippe 14, 15 in die Aufnahmeöffnung 12, 13 einmündet, und zwar im geradlinigen Teil der U-förmigen Aufnahmeöffnung 12, 13 dicht benachbart neben dem sich daran anschließenden bogenförmigen Teil der Aufnahmeöffnung 12, 13. In dieser Durchgangsbohrung 16 ist ein aus dieser Aufnahmeöffnung 12, 13 hervorstehender Druckkörper 17 längsverschieblich gelagert, an dem sich eine in der Aufnahmebohrung 12, 13 angeordnete Druckfeder 18 befindet, die sich auf der anderen Seite an einer in die Aufnahmeöffnung 12, 13 eingeschraubten Verschlußschraube 19 abstützt. Der Druckkörper 17 ist somit elastisch in die Aufnahmeöffnung 12, 13 einschiebbar und wird durch die Druckfeder 18 aus der Durchgangsbohrung 16 in die Aufnahmeöffnung 12, 13 vorgeschoben, dabei wird der Druckkörper 17 durch einen Anschlag 20 daran gehindert, aus der Durchgangsbohrung 16 vollständig auszutreten.

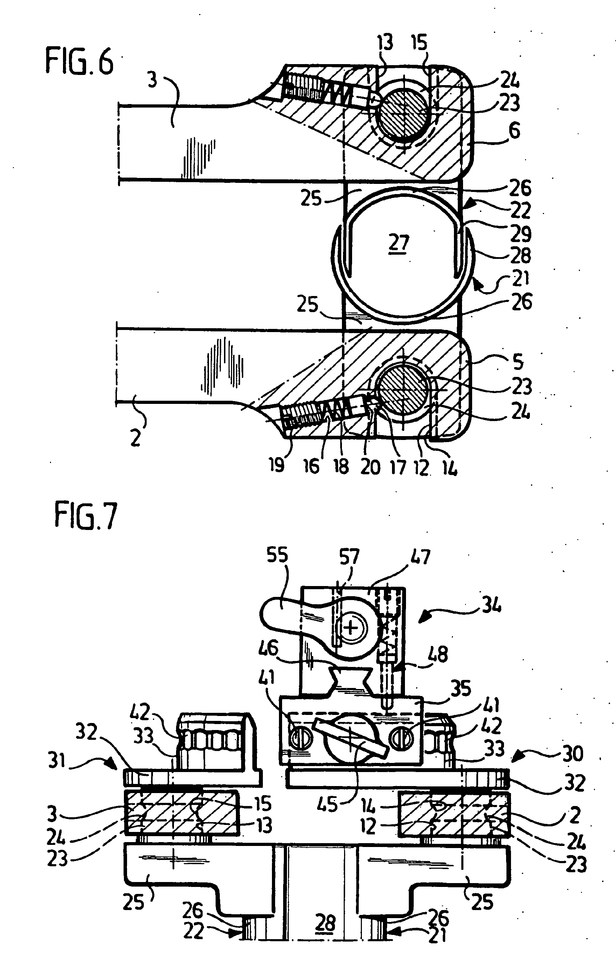

Die Aufnahmeöffnungen 12, 13 dienen der Aufnahme von zwei Retraktorblättern 21, 22, die mittels Haltezapfen 23 in diese Aufnahmeöffnungen 12, 13 einsetzbar sind. Diese Haltezapfen 23, die bei beiden Retraktorblättern 21 und 22 gleich ausgebildet sind, haben einen Durchmesser, der der Breite der Aufnahmeöffnungen 12, 13 entspricht und weisen eine Umfangsnut 24 auf, in die beim seitlichen Einschieben der Haltezapfen 23 in die Aufnahmeöffnungen 12, 13 die Halterippen 14, 15 eintreten. Beim vollständigen Einschieben der Haltezapfen 23 werden diese in den Aufnahmeöffnungen 12, 13 durch die Druckkörper 17 festgelegt, die sich an die Außenwand der Haltezapfen 23 anlegen und diese in die Aufnahmeöffnungen 12, 13 einschieben (Figur 6). Unter Zusammendrücken der Druckfeder 18 kann jedoch der Haltezapfen 23 wieder aus den Aufnahmeöffnungen 12, 13 herausgezogen werden, dazu benötigt der Operateur einen gewissen Kraftaufwand, so daß ein unbeabsichtigtes Austreten der Haltezapfen 23 aus den Aufnahmeöffnungen 12, 13 verhindert wird.

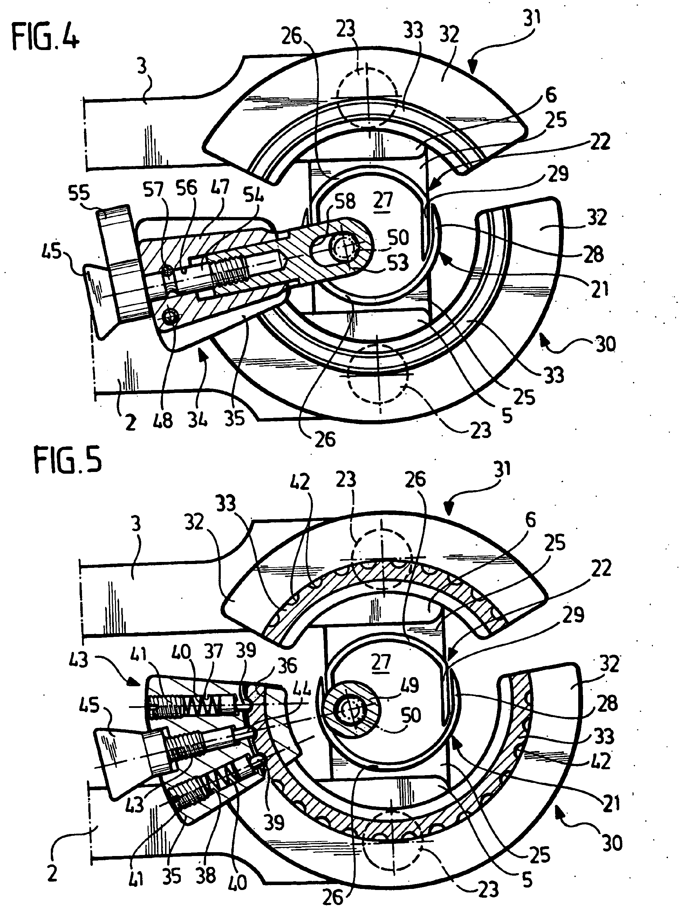

Die Haltezapfen 23 sind jeweils über ein Verbindungsteil 25 mit einem parallel zu den Haltezapfen 23 von dem Verbindungsteil 25 nach unten abstehenden Rückhalteelement 26 verbunden, welches aus einer halbkreisförmig gebogenen Wand besteht, die an ihrem unteren Ende geringfügig konisch zusammenläuft. Die beiden Rückhalteelemente 26 sind im eingesetzten Zustand jeweils zum gegenüberliegenden Rückhalteelement 26 offen und bilden gemeinsam mit diesem einen Zugangskanal 27 aus. Beide Rückhalteelemente 26 enden in Randbereichen 28 bzw. 29, die parallel zueinander verlaufen und außerdem parallel zur Verschieberichtung der Aufnahmeöffnungen 12, 13 beim Öffnen und Schließen der Haltearme 2, 3 durch Verdrehen der Lagerschraube 4. Die Randbereiche 28 und 29 überdecken sich dabei, so daß bei der Vergrößerung des Abstandes der beiden Rückhalteelemente 26 der von ihnen eingeschlossene Zugangskanal 27 über einen größeren Bereich allseits geschlossen bleibt (Figuren 4 bis 6).

Jeder der beiden Haltezapfen 23 trägt an seinem dem Verbindungsteil 25 gegenüberliegenden oberen Ende eine im wesentlichen halbkreisförmige Führung 30, 31, die ein horizontales, flanschartiges Basisteil 32 und ein senkrecht nach oben abstehendes, leistenförmiges Führungsteil 33 umfaßt. Dabei sind die Führungen 30 und 31 so ausgebildet, daß bei maximal angenäherten Retraktorblättern 21 und 22 die Führungsteile 33 einen im wesentlichen geschlossenen Ring ausbilden, welcher den Zugangskanal 27 im Abstand umgibt.

Die Führungsteile 33 verlaufen konzentrisch zu den Rückhalteelementen 26, weisen einen größeren Radius auf und sind ebenso wie die Rückhalteelemente 26 zueinander hin geöffnet.

Ein Halter 34 weist ein Unterteil 35 mit einer nach unten hin offenen Führungsnut 36 auf, die entsprechend dem Führungsteil 33 ausgebildet ist und die das Führungsteil 33 in sich aufnimmt, wenn der Halter 34 mit dem Unterteil 35 von oben her auf das Führungsteil 33 eines Retraktorblattes 21, 22 aufgesetzt wird. Ein in dieser Weise aufgesetztes Unterteil 35 kann längs des Führungsteiles 33 verschoben werden, und es sind geeignete Mittel vorgesehen, um den Halter 34 in verschiedenen Positionen längs des Führungsteils 33 festzulegen.

Dazu sind im Unterteil 35 in Durchgangsbohrungen 37 und 38, die radial von außen her in die Führungsnut 36 einmünden, Druckstücke 39 in Längsrichtung verschiebbar gelagert, die von einer in der Durchgangsbohrung 37, 38 angeordneten Druckfeder 40 in Richtung auf die Führungsnut 36 verschoben werden. Die Druckfeder 40 stützt sich jeweils an einer Verschlußschraube 41 ab, die in die Durchgangsbohrungen 37, 38 eingeschraubt ist, und die Druckstücke 39 tauchen unter der Wirkung der Druckfeder 40 in jeweils eine von mehreren Vertiefungen 42 auf der Außenseite des leistenförmigen Führungsteiles 33 ein, die in Umfangsrichtung verteilt sind. Dadurch ergibt sich eine elastische Rasterung bei der Verschiebung des Unterteiles 35 auf dem Führungsteil 33, bei Aufbringung einer bestimmten Verschiebekraft kann der Halter 34 gerastert längs des Führungsteiles 33 verschoben werden, wird aber bei einer erreichten Endposition in dieser gehalten.

Eine dauerhafte Fixierung kann erfolgen durch eine Schraubspindel 43, die zwischen den beiden Durchgangsbohrungen 37 und 38 in radialer Richtung in das Unterteil 35 eingeschraubt ist und mit ihrem freien Ende 44 in eine Vertiefung 42 des Führungsteils 33 eintauchen kann. Die Schraubspindel 43 ist mittels eines Griffes 45 verdrehbar und kann den Halter 34 in einer bestimmten Position längs des Führungsteils 33 dauerhaft festlegen.

Bei gelöster Schraubspindel 43 läßt sich das Unterteil 35 längs des Führungsteils 33 gerastert verschieben und kann bei angenäherten Retraktorblättern 21, 22 auch auf das Führungsteil 33 des benachbarten Retraktorblattes geschoben werden, wenn die Führungsteile 33 genau ringförmig zueinander angeordnet sind. Im übrigen ist es auch möglich, das Unterteil 35 nach Lösen der Schraubspindel 43 nach oben abzuziehen und von oben her auf das andere Führungsteil 33 aufzustecken.

Am Unterteil 35 des Halters 34 ist auf einer radial zum Führungsteil 33 angeordneten Führung 46 eine Halterung 47 schlittenartig verschieblich gelagert, deren Verschiebebewegung ebenfalls gerastert erfolgt, und zwar durch ein elastisches Rastelement, welches in eine Ausnehmung des Unterteils 35 eintritt. Diese Rasterung kann in gleicher Weise erreicht werden, wie im Falle der Verschiebung des Unterteils 35 längs des Führungsteils 33, also mittels eines Druckstückes, das mit einer Druckfeder gegen eine Vertiefung des Unterteils gespannt wird, dies ist in Figur 7 durch das Bezugszeichen 48 schematisch angedeutet.

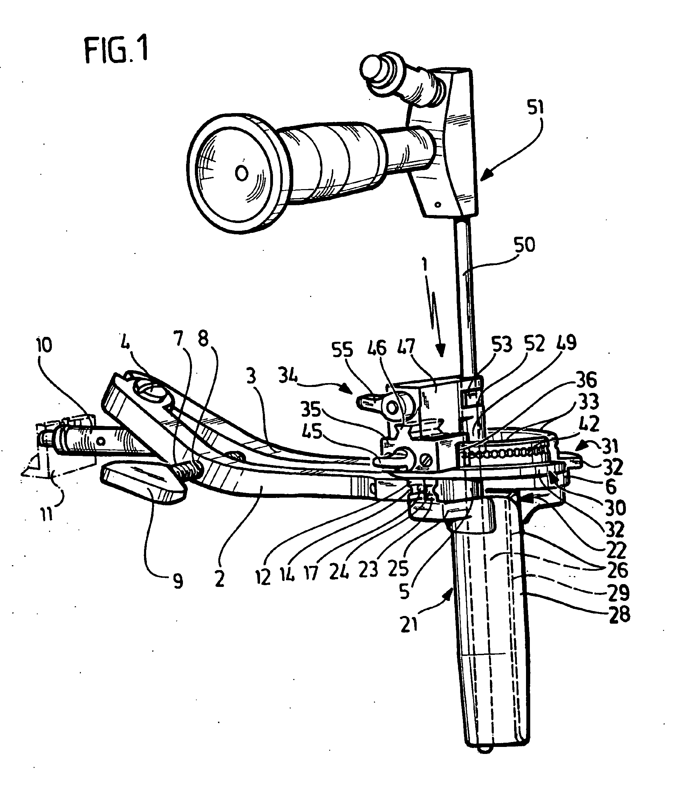

Die Halterung 47 weist eine Aufnahmehülse 49 auf, deren Längsachse parallel zur Längsachse des Zugangskanals 27 verläuft und die als Aufnahme für einen Rohrschaft 50 eines chirurgischen Instruments 51 dient, im dargestellten Ausführungsbeispiel der Figur 1 eines Endoskops. In einem quer zur Längsrichtung der Aufnahmehülse 49 angeordneten und die Aufnahmehülse 49 durchsetzenden Schlitz 52 der Halterung 47 ist ein hülsenförmiges Spannelement 53 parallel zur Verschieberichtung der Halterung 47 auf dem Unterteil 35 verschieblich gelagert, in dieses Spannelement 53 ist eine Spannschraube 54 parallel zu dieser Verschieberichtung eingeschraubt, die über ein Griffteil 55 verdreht werden kann. Die Spannschraube 54 ist in einer Bohrung 56 der Halterung 47 drehbar und durch einen Stift 37 axial unverschiebbar gelagert, so daß bei einer Verdrehung der Spannschraube 54 das Spannelement 53 parallel zur Verschieberichtung der Halterung 47 in derselben verschoben werden kann.

Das Spannelement 53 weist eine Durchbrechung 58 auf in Form eines Langloches, dessen Längsachse parallel zur Verschieberichtung des Spannelementes 53 verläuft und dessen Breite dem Innendurchmesser der Aufnahmehülse 49 entspricht. Diese Durchbrechung 58 überdeckt den Innenraum der Aufnahmehülse 49, so daß ein durch die Aufnahmehülse 49 geschobener Rohrschaft 50 die Durchbrechung 58 des Spannelementes 53 durchsetzt. Verschiebt man das Spannelement 53 mittels der Spannschraube 54, so spannt dadurch das Spannelement 53 den Rohrschaft gegen die Wand der Aufnahmehülse 49 und legt dadurch den Rohrschaft in der Aufnahmehülse 49 gegen Drehung und axiale Verschiebung fest. Diese Klemmverbindung ist durch Lösen der Spannschraube 54 jederzeit lösbar.

Bei der Benutzung der beschriebenen Vorrichtung wird diese normalerweise zunächst ohne den Halter 34 und ohne Instrument 51 verwendet. Ein Körperzugang wird beispielsweise über einen in den Körper eingeführten Draht und über eine oder mehrere konzentrisch darüber geschobene Hülsen eröffnet, und über eine solche Hülse werden die beiden Rückhalteelemente 56 in das Innere des Körpers eingeschoben, sie bilden dabei ein über das in den Körper eingebrachte Rohr geschobenes Rohr aus und verdrängen das Körpergewebe seitlich. Nach Entfernung des zuerst eingesetzten Drahtes und der zuerst eingesetzten Rohre definieren die beiden Rückhalteelemente 26 somit einen rohrförmigen Zugang in das Innere des Körpers, dessen Größe durch Verschwenken der Haltearme vergrößert werden kann, dazu genügt es, die Verstellschraube 7 zu betätigen. Der Zugangskanal 27 geht dabei bei der Vergrößerung von einem kreisförmigen Querschnitt in einen im wesentlichen ovalen Querschnitt über.

Nach diesem Einsetzen der Vorrichtung und der gewünschten Wahl des Querschnittes des Zugangskanals 27 wird der Halter 34 auf eine der Führungen 30 oder 31 von oben her aufgesetzt und durch Verschiebung längs der Führungsteile 33 in die gewünschte Winkellage verschwenkt. Das gewünschte Instrument 51 wird dann mit dem Rohrschaft 50 durch die Aufnahmehülse 49 eingeschoben, bis die gewünschte Einschubtiefe erreicht ist. Diese Position kann mittels der Spannschraube 54 festgelegt werden, falls die Position des Rohrschaftes 50 im Querschnitt des Zugangskanals 27 noch geändert werden soll, ist dies durch Verschiebung des Halters 34 längs des Führungsteiles 33 und durch Verschiebung der Halterung 47 relativ zum Unterteil 35 möglich. Bei Erreichen der gewünschten Endposition wird die Schraubspindel 53 festgezogen, so daß eine weitere Verschiebung längs des Führungsteils 33 nicht mehr möglich ist.

Auf diese Weise kann der Rohrschaft 50 an jeder beliebigen Stelle des Querschnitts des Zugangskanals 27 positioniert werden, und es ist auch in einfachster Weise möglich, diese Position den jeweiligen Wünschen anzupassen und zu verändern.

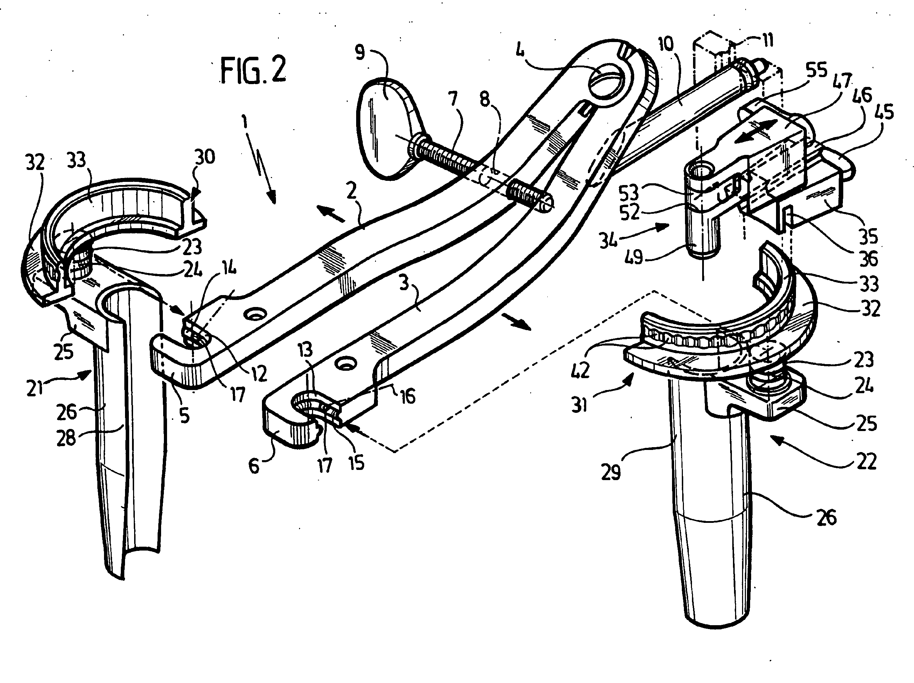

Eine Zerlegung des Instrumentes ist in einfachster Weise möglich, es genügt dazu, die Spannschraube 54 und die Schraubspindel 43 zu lösen, dann lassen sich die Teile in der in Figur 2 deutlich sichtbaren Weise voneinander trennen und getrennt reinigen. Insbesondere ist es auch möglich, ganze Retraktorblätter auszutauschen, wenn dies gewünscht wird.

Der Querschnitt des Zugangskanals 27 kann während der gesamten Operation jederzeit durch Verdrehen der Verstellschraube 27 geändert werden, dies ist auch möglich, wenn ein Instrument 51 in den Zugangskanal 27 eingesetzt ist.

Durch die variable Verschiebbarkeit des Halters 34 ist insbesondere sichergestellt, daß der Rohrschaft 50 innerhalb des Zugangskanals 27 immer an einer Stelle angeordnet werden kann, die einen weiteren Zugang durch den Zugangskanal 27 zur Operationsstelle ermöglicht und diesen Zugang nicht behindert. Der Durchmesser des Rohrschaftes 50 kann beispielsweise in der Größenordnung von 7 mm liegen, der Durchmesser der Rückhalteelemente 26 dagegen in der Größenordnung von 15 mm, so daß die Querschnittsfläche des Rohrschaftes 50 erheblich kleiner ist als die Querschnittsfläche des Zugangskanals 27.

## Patentansprüche

1. Vorrichtung (1) zur Schaffung eines perkutanen Zugangs in einen Körper mit zwei gemeinsam einen Zugangskanal (27) bildenden, jeweils ein proximales und ein distales Ende aufweisenden Retraktorblättern (21, 22), deren Abstand voneinander unter Veränderung des Querschnitts des Zugangskanals (27) veränderbar ist und an deren proximalem Ende je eine Führung (30, 31) angeordnet ist, auf der ein Halter (34) für ein einen Schaft (50) aufweisendes Instrument (51) derart in einer quer zum Zugangskanal (27) angeordneten Ebene verschiebbar gelagert ist, daß ein am Halter (34) gehaltenes Instrument (51) mit seinem Schaft (50) an verschiedener Stelle des Querschnitts des Zugangskanals (27) in diesen hineintaucht, **dadurch gekennzeichnet, daß** die Führungen (30, 31) parallel zur Kontur der Retraktorblätter (26) verlaufen und dass die Führungen (30, 31) der beiden Retraktorblätter (21, 22) bei maximal angenäherten Retraktorblättern (21, 22) den Zugangskanal (27) ringförmig umgeben.

2. Vorrichtung nach Anspruch 1, **dadurch gekennzeichnet, daß** die die Führungen (30, 31) kreisbogenförmig verlaufen.

3. Vorrichtung nach einem der voranstehenden Ansprüche, **dadurch gekennzeichnet, daß** die Führungen (30, 31) als in proximaler Richtung von den Retraktorblättern (21, 22) abstehende Leisten (33) ausgebildet sind.

4. Vorrichtung nach einem der voranstehenden Ansprüche, **dadurch gekennzeichnet, daß** der Halter (34) eine Fixiereinrichtung (43, 44, 45) aufweist, mittels der er relativ zu der Führung (33) lösbar fixierbar ist.

5. Vorrichtung nach Anspruch 4, **dadurch gekennzeichnet, daß** die Fixiereinrichtung (43, 44, 45) eine Klemmeinrichtung ist.

6. Vorrichtung nach einem der voranstehenden Ansprüche, **dadurch gekennzeichnet, daß** die Führung (33) und der Halter (34) zusammenwirkende Rastelemente (39, 42) tragen, durch die der Halter (34) gerastert längs der Führung (33) verschiebbar ist.

7. Vorrichtung nach Anspruch 6, **dadurch gekennzeichnet, daß** die Rastelemente Vertiefungen (42) und elastisch in diese eingreifende Vorsprünge (39) sind.

8. Vorrichtung nach einem der voranstehenden Ansprüche, **dadurch gekennzeichnet, daß** der Halter (34) eine Halterung (47) für das Instrument (51) trägt, die relativ zum Halter (34) in Richtung auf den Mittelpunkt des Zugangskanals (27) verschiebbar ist.

9. Vorrichtung nach Anspruch 8, **dadurch gekennzeichnet, daß** die Halterung (47) an einem Schlitten angeordnet ist, der an einer Führung (46) des Halters (34) verschiebbar gelagert ist.

10. Vorrichtung nach Anspruch 8 oder 9, **dadurch gekennzeichnet, daß** die Halterung (47) relativ zum Halter (34) in verschiedenen Positionen lösbar fixierbar ist.

11. Vorrichtung nach Anspruch 10, **dadurch gekennzeichnet, daß** Halter (34) und Halterung (47) durch elastisch zusammenwirkende Rastelemente (48) gerastert gegeneinander verschiebbar sind.

12. Vorrichtung nach einem der voranstehenden Ansprüche, **dadurch gekennzeichnet, daß** am Halter (34) eine Spannvorrichtung (49, 53) zur lösbaren Fixierung des Instruments (51) am Halter (34) angeordnet ist.

13. Vorrichtung nach Anspruch 12, **dadurch gekennzeichnet, daß** die Spannvorrichtung zwei nebeneinander den Schaft (50) des Instruments (51) umgebende Halteglieder (49, 53) aufweist, die in einer quer zur Längsachse des Schaftes (50) verlaufenden Ebene gegeneinander verschiebbar sind.

14. Vorrichtung nach einem der voranstehenden Ansprüche, **dadurch gekennzeichnet, daß** die Retraktorblätter (26) im Querschnitt bogenförmig ausgebildet sind und in seitliche Randbereiche (28, 29) auslaufen, die parallel zu ihrer Verschiebung beim Verändern des gegenseitigen Abstandes verlaufen und sich bei angenäherten Retraktorblättern (21, 22) überdecken.

15. Vorrichtung nach einem der voranstehenden Ansprüche, **dadurch gekennzeichnet, daß** die Retraktorblätter (21, 22) an gegeneinander bewegbaren und **dadurch** den Abstand der Retraktorblätter (21, 22) voneinander verändernden Tragelementen (2, 3) gehalten sind.

16. Vorrichtung nach Anspruch 15, **dadurch gekennzeichnet, daß** die Tragelemente zwei Haltearme (2, 3) sind, die schwenkbar miteinander verbunden sind und an ihren freien Enden (5, 6) jeweils ein Retraktorblatt (21 bzw. 22) tragen.

17. Vorrichtung nach einem der Ansprüche 15 oder 16, **dadurch gekennzeichnet, daß** die Tragelemente (2, 3) an einer ortsfesten Rahmenstruktur (11) festlegbar sind.

18. Vorrichtung nach einem der Ansprüche 15 bis 17, **dadurch gekennzeichnet, daß** die Tragelemente (2, 3) durch eine Feststellvorrichtung (7, 8, 9) in festem Abstand zueinander fixierbar sind.

19. Vorrichtung nach einem der Ansprüche 15 bis 18, **dadurch gekennzeichnet daß** die Retraktorblätter (21, 22) an den Tragelementen (2, 3) lösbar gehalten sind.

20. Vorrichtung nach Anspruch 19, **dadurch gekennzeichnet, daß** zur lösbaren Festlegung der Retraktorblätter (21, 22) an den Tragelementen (2, 3) zusammenwirkende elastische Rastelemente (23, 17) vorgesehen sind.

21. Vorrichtung nach einem der voranstehenden Ansprüche, **dadurch gekennzeichnet, daß** das im Halter (34) gehaltene Instrument (51) ein Endoskop ist.

## Claims

1. Device (1) for creating a percutaneous access in a body with two retractor blades (21, 22) together forming an access channel (27) and each having a proximal and a distal end, the spacing of the retractor blades from one another being variable by changing the cross-section of the access channel (27) and a respective guide (30, 31) being formed on the proximal ends thereof, on which a holder (34) for an instrument (51) having a shaft (50) is displaceably mounted in a plane arranged transversely to the access channel (27) in such a way that the shaft (50) of an instrument (51), which is held on the holder (34), enters the access channel (27) at various points of the cross-section of the access channel (27), **characterised in that** the guides (30, 31) extend parallel to the contour of the retractor blades (26) and **in that** the guides (30, 31) of the two retractor blades (21, 22) annularly surround the access channel (27) when the retractor blades (21, 22) are closest together.

2. Device according to claim 1, **characterised in that** the guides (30, 31) extend in the manner of a circular arc.

3. Device according to any one of the preceding claims, **characterised in that** the guides (30, 31) are configured as strips (33) projecting from the retractor blades (21, 22) in the proximal direction.

4. Device according to any one of the preceding claims, **characterised in that** the holder (34) has a fixing mechanism (43, 44, 45), by means of which it can be detachably fixed relative to the guide (33).

5. Device according to claim 4, **characterised in that** the fixing mechanism (43, 44, 45) is a clamping mechanism.

6. Device according to any one of the preceding claims, **characterised in that** the guide (33) and the holder (34) carry cooperating detent elements (39, 42), by means of which the holder (34) can be displaced in a latched manner along the guide (33).

7. Device according to claim 6, **characterised in that** the detent elements are indentations (42) and projections (39) engaging in a resilient manner therein.

8. Device according to any one of the preceding claims, **characterised in that** the holder (34) carries a retaining means (47) for the instrument (51), which can be displaced relative to the holder (34) in the direction of the centre point of the access channel (27).

9. Device according to claim 8, **characterised in that** the retaining means (47) is arranged on a slide, which is displaceably mounted on a guide (46) of the holder (34).

10. Device according to claim 8 or 9, **characterised in that** the retaining means (47) can be detachably fixed in various positions relative to the holder (34).

11. Device according to claim 10, **characterised in that** the holder (34) and retaining means (47) can be displaced relative to one another in a latched manner by resiliently cooperating latching elements (48).

12. Device according to any one of the preceding claims, **characterised in that** a clamping device (49, 53) is arranged on the holder (34) for the detachable fixing of the instrument (51) on the holder (34).

13. Device according to claim 12, **characterised in that** the clamping device has two mutually adjacent holding members (49, 53) which surround the shaft (50) of the instrument (51) and can be displaced relative to one another in a plane extending transverse to the longitudinal axis of the shaft (50).

14. Device according to any one of the preceding claims, **characterised in that** the retractor blades (26) are arcuate in cross-section and end in lateral edge regions (28, 29), which extend parallel to their displacement when the mutual spacing is changed and overlap one another when the retractor blades (21, 22) have moved close to one another.

15. Device according to any one of the preceding claims, **characterised in that** the retractor blades (21, 22) are held on carrying elements (2, 3) which can be moved relative to one another and thereby change the spacing of the retractor blades (21, 22) from one another.

16. Device according to claim 15, **characterised in that** the carrying elements are two holding arms (2, 3), which are pivotably connected to one another and at their free ends (5, 6) carry a respective retractor blade (21 or 22).

17. Device according to either of claims 15 or 16, **characterised in that** the carrying elements (2, 3) can be fixed to a stationary frame structure (11).

18. Device according to any one of claims 15 to 17, **characterised in that** the carrying elements (2, 3) can be fixed at a fixed spacing in relation to one another by a fixing device (7, 8, 9).

19. Device according to any one of claims 15 to 18, **characterised in that** the retractor blades (21, 22) are detachably held on the carrying elements (2, 3).

20. Device according to claim 19, **characterised in that** cooperating resilient latching elements (23, 17) are provided for the detachable fixing of the retractor blades (21, 22) on the carrying elements (2, 3).

21. Device according to any one of the preceding claims, **characterised in that** the instrument (51) held in the holder (34) is an endoscope.

## Revendications

1. Dispositif (1) pour réaliser un accès percutané dans un corps, comprenant deux lames d'écarteur (21,22) qui forment en commun un canal d'accès (27) et présentent chacune une extrémité proximale et une extrémité distale, dont la distance d'espacement de l'une à l'autre peut être modifiée en produisant ainsi une variation de la section transversale du canal d'accès (27), et à l'extrémité proximale desquelles est respectivement agencé un guide (30, 31) sur lequel est monté un support (34) pour un instrument (51) à corps allongé (50), en pouvant coulisser dans un plan agencé transversalement au canal d'accès (27), de façon à ce qu'un instrument (51) maintenu dans le support (34) s'engage avec son corps allongé (50) dans le canal d'accès (27) en des endroits différents de la section transversale de celui-ci, **caractérisé en ce que** les guides (30, 31) s'étendent parallèlement au contour des lames d'écarteur (21, 22), et **en ce que** les guides (30, 31) des deux lames d'écarteur (21, 22) entourent le canal d'accès (27) selon une forme annulaire lorsque les lames d'écarteur (21, 22) sont rapprochées au maximum l'une de l'autre.

2. Dispositif selon la revendication 1, **caractérisé en ce que** les guides (30, 31) s'étendent en forme d'arc de cercle.

3. Dispositif selon l'une des revendications précédentes, **caractérisé en ce que** les guides (30, 31) sont réalisés sous la forme de listels (33) faisant saillie des lames d'écarteur (21, 22) en direction proximale.

4. Dispositif selon l'une des revendications précédentes, **caractérisé en ce que** le support (34) comporte un système de fixation (43, 44, 45) au moyen duquel il peut être fixé de manière amovible par rapport au guide (33).

5. Dispositif selon la revendication 4, **caractérisé en ce que** le système de fixation (43, 44, 45) est un dispositif de blocage.

6. Dispositif selon l'une des revendications précédentes, **caractérisé en ce que** le guide (33) et le support (34) portent des éléments d'encliquetage (39, 42) interagissant mutuellement, par l'intermédiaire desquels le support (34) peut coulisser de manière encliquetable le long du guide (33).

7. Dispositif selon la revendication 6, **caractérisé en ce que** les éléments d'encliquetage sont des creux (42) et des protubérances (39) pouvant s'y engager élastiquement.

8. Dispositif selon l'une des revendications précédentes, **caractérisé en ce que** le support (34) porte un système de tenue (47) pour l'instrument (51), qui peut coulisser par rapport au support (34) en direction du centre du canal d'accès (27).

9. Dispositif selon la revendication 8, **caractérisé en ce que** le système de tenue (47) est agencé sur un chariot qui est monté coulissant sur un guide (46) du support (34).

10. Dispositif selon la revendication 8 ou la revendication 9, **caractérisé en ce que** le système de tenue (47) peut être fixé de manière amovible, dans différentes positions, par rapport au support (34).

11. Dispositif selon la revendication 10, **caractérisé en ce que** le support (34) et le système de tenue (47) peuvent coulisser l'un par rapport à l'autre de manière encliquetable grâce à des éléments d'encliquetage (48) interagissant mutuellement de manière élastique.

12. Dispositif selon l'une des revendications précédentes, **caractérisé en ce que** sur le support (34) est agencé un dispositif de serrage (49, 53) pour la fixation amovible de l'instrument (51) au support (34).

13. Dispositif selon la revendication 12, **caractérisé en ce que** le dispositif de serrage comprend deux organes de tenue (49, 53) qui entourent côte à côte le corps allongé (50) de l'instrument (51), et peuvent coulisser l'un par rapport à l'autre dans un plan s'étendant transversalement à l'axe longitudinal du corps allongé (50).

14. Dispositif selon l'une des revendications précédentes, **caractérisé en ce que** les lames d'écarteur (26) sont d'une configuration courbe en section transversale, et se terminent par des zones de bordure latérales (28, 29), qui s'étendent parallèlement à leur coulissement lorsque l'on fait varier la distance d'espacement réciproque, et se chevauchent mutuellement lorsque les lames d'écarteur (21, 22) sont rapprochées.

15. Dispositif selon l'une des revendications précédentes, **caractérisé en ce que** les lames d'écarteur (21, 22) sont tenues sur des éléments de support (2, 3) mobiles l'un à l'encontre de l'autre et faisant ainsi varier la distance d'espacement des lames d'écarteur (21, 22) l'une par rapport à l'autre.

16. Dispositif selon la revendication 15, **caractérisé en ce que** les éléments de support sont deux bras de support (2, 3), qui sont reliés mutuellement de manière pivotante et portent à leurs extrémités libres (5, 6) respectivement une lame d'écarteur (21 respectivement 22).

17. Dispositif selon la revendication 15 ou la revendication 16, **caractérisé en ce que** les éléments de support (2, 3) peuvent être fixés sur une structure de cadre (11) en position fixe.

18. Dispositif selon l'une des revendications 15 à 17, **caractérisé en ce que** les éléments de support (2, 3) peuvent être fixés à une distance d'espacement fixe l'un de l'autre, grâce à un dispositif d'immobilisation (7, 8, 9).

19. Dispositif selon l'une des revendications 15 à 18, **caractérisé en ce que** les lames d'écarteur (21, 22) sont tenues de manière amovible sur les éléments de support (2, 3).

20. Dispositif selon la revendication 19, **caractérisé en ce que** pour la fixation amovible des lames d'écarteur (21, 22) aux éléments de support (2, 3), sont prévus des éléments d'encliquetage élastiques (23, 17) interagissant mutuellement.

21. Dispositif selon l'une des revendications précédentes, **caractérisé en ce que** l'instrument (51) tenu dans le support (34) est un endoscope.
